# EUROPÄISCHE PATENTSCHRIFT

(11) **EP 1 567 115 B1**
(45) Veröffentlichungstag und Bekanntmachung des Hinweises auf die Patenterteilung: **13.09.2006**
(21) Anmeldenummer: 03778319.8
(22) Anmeldetag: 17.11.2003
(51) Int. Cl.: A61K 8/00, C08L 33/14, C08L 33/26

(54) **ZUSAMMENSETZUNGEN, DIE MINDESTENS EIN COPOLYMER (A) UND MINDESTENS EIN COPOLYMER (B) ENTHALTEN, UND DEREN VERWENDUNG IN KOSMETISCHEN ZUBEREITUNGEN**
COMPOSITIONS COMPRISING AT LEAST ONE COPOLYMER (A) AND AT LEAST ONE COPOLYMER (B), AND USE THEREOF IN COSMETIC PREPARATIONS
COMPOSITIONS CONTENANT AU MOINS UN COPOLYMERE (A) ET AU MOINS UN COPOLYMERE (B) ET LEUR UTILISATION DANS DES PREPARATIONS COSMETIQUES

(30) Priorität: 29.11.2002 DE 10256148
(43) Veröffentlichungstag der Anmeldung: 31.08.2005
(73) Patentinhaber: BASF Aktiengesellschaft, 67056 Ludwigshafen (DE)
(72) Erfinder: MANTECA ZUAZO, Izaskun, 67063 Ludwigshafen (DE); GOMEZ, Marcos, 69117 Heidelberg (DE); NGUYEN-KIM, Son, 69502 Hemsbach (DE)
(86) Internationale Anmeldenummer: PCT/EP2003/012816
(87) Internationale Veröffentlichungsnummer: WO 2004/050043

(56) Entgegenhaltungen:
- EP-A- 0 824 914
- DE-A- 10 113 227

## Beschreibung

Bei kosmetischen Zubereitungen, insbesondere solchen auf Öl, Creme oder Lotionbasis sind Eigenschaften wie gutes Hautgefühl, Wasserbindungsvermögen und Absorption, Konsistenz und Einziehvermögen entscheidende Größen. Diese Eigenschaften werden durch das physikalische Verhalten der kosmetischen Zubereitungen unter der Einwirkung von mechanischen Kräften beeinflusst. Das rheologische Profil einer kosmetischen Zubereitung ist für die genannten Eigenschaften von großer Bedeutung.

Zur Beeinflussung des rheologischen Profils werden kosmetischen Zubereitungen beispielsweise nicht-wasserlösliche polymere Verdicker zugesetzt. Als verdickende Zusätze sind beispielsweise Fettalkoholethoxylate, Fettsäurealkylolamide, ethoxylierte Glucoseester, ethoxylierte Partialglyceride oder Kochsalz bekannt. Zuviel Kochsalz kann in einer auf Fettalkoholethersulfat basierenden Formulierung aber in das Gegenteil umschlagen und das Produkt wir immer niedriger viskos und im Extremfall kommt es zu einer Phasenseparation.

Eine andere Möglichkeit der Verdickung besteht darin, die viskositätserhöhende Wirkung von Tensidgemischen auszunutzen. Alkylbetaine und Alkylpolyglykoside verdicken in geringen Zusätzen Formulierungen auf Basis Fettalkoholethersulfat.

Strukturviskose Produkte mit Gelcharakter, die sich beispielsweise zur Anwendung aus der Tube eignen, werden durch Zusatz von polymeren Gelbildnern, z.B. Polyacrylsäure, Xanthan oder Hydroxypropylmethylcellulose, erhalten.

**EP 0 824 914 B1** beschreibt ein verdicktes Körperpflegemitteln, welches einen polymeren Rheologiemodifizierer enthält. Dieser ist erhältlich durch Polymerisation von C1-C6- Alkyl(meth)acrylsäureestern, (Methy)acrylamide und sog. assoziativen Monomeren.

**WO 97/36572** beschreibt eine kosmetische Zubereitung, welche mindestens ein Silikonöl und ein Verdicker enthält, wobei der Verdicker ein Polymer ist, welches Silikongruppen und als weitere funktionelle Gruppen beispielsweise Harnstoffgruppen enthält.

Aufgabe der vorliegenden Erfindung bestand darin, neue Polymere zur Verfügung zu stellen, welche sich als Verdicker, insbesondere als Verdicker in kosmetischen Zubereitungen, eignen. Von besonderem Interesse waren kosmetische Zubereitungen, die eine Ölphase enthalten.

**DE 34 43 964 A1** und **DE 39 02 103 A1** beschreiben Bindemittel für wässrige Glanzfarben, welche aus mehrphasigen Emulsionspolymerisaten bestehen. Diese sind aus einem harten Kernmaterila und einem weichen Schalenmaterial aufgebaut. Am Aufbau des Schalenmaterials können Monomere mit Ureidogruppen beteiligt sein.

**DE 39 02 555 A1** beschreibt wässrige Polyacrylat Systeme für die Lackierung von Kunststoffoberflächen, welche bis zu 10 Gew.-% einer polymerisierbaren Ureidoverbindung enthalten können, wie beispielsweise N-Methacrylamidomethylenharnstoff, N-(2-Methacryloyloxyethyl)ethylenharnstoff, und N-Vinylimidazol.

**DE 39 02 067 A1** beschreibt filmbildende, selbstvernetzende wässrige Kunststoffdispersionen enthaltend ein Emulsionspolymerisat, welches ein radikalisch polymerisierbares Monomer mit einer Ureidogruppe enthalten kann. Verwendet werden diese Dispersionen zum Anstrich von Kunststoffoberflächen.

**DE 43 34 178 A1** (= EP 722 477 A1, WO 95/09896 A1) beschreibt wässrige Polymerzubereitungen, die ein Polymerisat, das Ureidogruppen aufweist, und eine Polyaldehydverbindung enthalten. Verwendet werden diese Zubereitungen zur Herstellung von Beschichtungen mit erhöhter Haftung am Substrat sowie mit erhöhter innerer Festigkeit.

**DE 100 41 680 A1** beschreibt wässrige Polymerisatdispersionen, welche als Monomeren Harnstoffderivate, wie z.B. N-(2-Methacryloxyethyl)ethylenharnstoff oder N-(2-Methyacryloxyethyl)thioharnstoff bis zu 5 Gew.-% enthalten können. Diese Dispersionen eignen als Bindemittel in Beschichtungsmitteln und gewährleisten hohen Glanz und hohe Blockfestigkeit der Beschichtungsmittel.

**US 3,645,965** beschreibt synthetische Polymere, welche durch Einbau von Imidazolidin-4-onen erhöhte Lichtung und Temperaturbeständigkeit aufweisen.

**US 3,876,657** beschreibt die Herstellung von 1- substituierten Imidazolidin-2-onen und die Verwendung dieser Verbindungen als Monomere für Polymere und Copolymere.

**US 5,852,123** beschreibt Propfpolymere enthaltend eine Harnstoff- oder Imidgruppe und die Verwendung dieser Pfropfpolymere zum Dispergieren von Pigmenten. Die Polymere bestehen zu 2 bis 97 Gew.-% aus einem Rückrat eines Polymers aus ethylenisch ungesättigten Monomeren, 97 bis 2 Gew.-% aus einem Makromer, welches über eine terminale, ethylenisch ungesättigte Gruppe auf das Rückrat gepfropft ist und mindestens 1 Gew.-% eines dispergierenden Agens, welche eine Imid-Gruppe und/oder eine Harnstoff-Gruppe enthält.

US 5,972,431 beschreibt wässrige Zubereitungen für Überzugsmittel, Tinten und landwirtschaftliche Zusammensetzungen, welche zur Verminderung der Oberflächenspannung ein zyklisches Harnstoffderivat enthalten.

EP 0 124 713 B1 beschreibt Imidazolidinon-Monomere sowie Polymere aus diesen Monomeren und ethylenisch ungesättigten Monomeren sowie die Verwendung dieser Polymere als Formaldehyd-freies Bindemittel für nicht gewebte Textilien.

**EP 0 609 793 A2** beschreibt eine wässriges Bindemittel, enthaltend eine filmbildende wässrige Kunststoffdispersion und eine Vernetzungskomponente. Die Kunststoffdispersion ist ein Emulsionspolymerisat mit polymerisierten Einheiten eines ethylenisch ungesättigten, radikalisch polymerisierbaren Monomeren mit einer seitenständigen Alkylenharnstoffgruppe. Die Vernetzungskomponente besteht aus polyfunktionellen Aldehyden.

**EP 1 000 610 A1** (DE 19850363) beschreibt die Verwendung von Polymeren, an die Derivate von Ethylenharnstoff gebunden sind in Kosmetika und als Haarfestigerpolymere. Diese Polymere zeichnen sich durch verbesserte Auswaschbarkeit bei herabgesetzter Lösungsviskosität und unveränderter Festigungswirkung aus

Gegenstand der vorliegenden Erfindung sind Zusammensetzungen, enthaltend
(A) Copolymer aus
   (A-1) mindestens einem ethylenisch ungesättigten, radikalisch copolymerisierbaren Monomeren der allgemeinen Formel (I)

      Y-NR¹-C(V)-NHR² (I),

      wobei die Substituenten folgende Bedeutung besitzen:
      - Y =: einem zur radikalischen Polymerisation befähigten ethylenisch ungesättigten Rest
      - V =: O, S oder NH
      - R¹, R² =: unabhängig voneinander H oder eine C₁- bis C₈-Alkylgruppe, oder beide zusammen eine überbrückende C₂- bis C₄-Alkylengruppe, die bis zu zweifach mit einer C₁- bis C₄-Alkoxygruppe und/oder Hydroxylgruppe substituiert sein kann,
   (A-2) mindestens einem ungesättigten Monomeren der allgemeinen Formel (II)

      X-C(O)CR⁷ = CHR⁶ (II),

      wobei die Substituenten folgende Bedeutung besitzen:
      X ausgewählt ist aus der Gruppe der Reste -OH, -OR⁸, NH₂, -NHR⁸, N(R⁸)₂;
      die Reste R⁸ können identisch oder verschieden ausgewählt werden aus der Gruppe, bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl;
      R⁷ und R⁶ sind unabhängig voneinander, ausgewählt aus der Gruppe, bestehend aus -H, C₁-C₈ linear- oder verzweigtkettige Alkylketten, Methoxy, Ethoxy, 2-Hydroxyethoxy, 2-Methoxyethoxy und 2-Ethoxyethyl.
(B) mindestens einem weiteren von (A) verschiedenem Copolymeren aus
   (B-1) mindestens einem Monomeren der allgemeinen Formel (III) 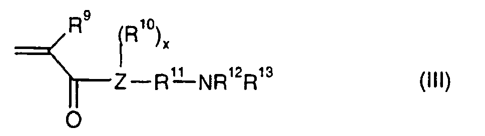 mit
      - R⁹: = H, Alkyl mit 1 bis 8 C-Atomen,
      - R¹⁰: = H, Methyl,
      - R¹¹: = Alkylen mit 1 bis 24 C-Atomen, optional substituiert durch C₁-C₆-Alkyl,
      - R¹², R¹³: = C₁-C₄₀ Alkylrest,
      - Z: = Stickstoff für x = 1 oder Sauerstoff für
      x = 0.
      und
   (B-2) mindestens einem ethylenisch ungesättigten Monomeren.

Als Monomer (A-1) geeignet sind insbesondere Verbindungen mit V = O, sogenannte Ureidoverbindungen.

Als Monomer (A-1) geeignet sind Verbindungen mit V = S, sogenannte Thioureidoverbindungen.

Als Monomer (A-1) geeignet sind Verbindungen mit V= NH, sogenannte Guanidinoverbindungen.

In einer bevorzugten Ausführungsform wird als Monomer (A-1) mindestens eine Verbindung der allgemeinen Formel (la) eingesetzt 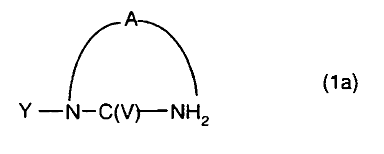 wobei A = eine 2 oder 3 gliedrige, gegebenenfalls eine Carbonylgruppe aufweisende Alkylengruppe darstellt.

Besonders bevorzugt sind Verbindungen der allgemeinen Formel (la), in der A eine unsubstituierte 2gliedrige Alkylengruppe darstellt.

Besonders bevorzugt sind Verbindungen der allgemeinen Formel (la), in der V = O.

In einer bevorzugten Ausführungsform bilden R¹ und R² zusammen eine, gegebenenfalls substituierte überbrückende C2-Alkylengruppe.

Besonders bevorzugt sind Verbindungen der allgemeinen Formel (la), in der A eine unsubstituierte 2gliedrige Alkylengruppe darstellt und in der V = O.

In einer bevorzugten Ausführungsform wird als Monomer (A-1) mindestens eine Verbindung der allgemeinen Formel (Ib) eingesetzt 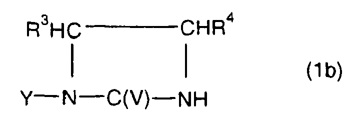 wobei R³ und R⁴ unabhängig voneinander für H, -OH, -NH, C₁- bis C₈-Alkyl stehen.

Besonders bevorzugt sind Verbindungen der allgemeinen Formel (Ib), in der V = O.
Besonders bevorzugt sind Verbindungen der allgemeinen Formel (Ib), in der R₃ und R₄ = H. Besonders bevorzugt sind Verbindungen der allgemeinen Formel (Ib), in der V=O, R₃ und R₄ = H und Y = CH₂=C(CH₃)-CO-O-(CH₂)₂.

Besonders bevorzugt sind Verbindungen der allgemeinen Formel (Ib), in der V=S, R₃ und R₄ = H und Y = CH₂=C(CH₃)-CO-O-(CH₂)₂-

Besonders bevorzugt sind Verbindungen der allgemeinen Formel (Ib), in der V=NH, R₃ und R₄ = H und Y = CH₂=C(CH₃)-CO-O-(CH₂)₂- (= Ureidomethacrylat).

Als Y in Formel (I) ist jeder zur radikalischen Polymersiation befähigte ethylenische ungesättigte Rest geeignet.

In einer bevorzugten Ausführungsform der vorliegenden Erfindung wird als ethylenische ungesättigter Rest Y ein Rest der folgenden Formel (IV) eingesetzt

Y = CH₂=CR⁵-CO-W-(CH₂)ₙ- (IV),

wobei
- R⁵: = H, CH₃
- W: = O, NH
- n: = 2 bis 8, insbesondere 2 bis 4
darstellt.

Beispielhaft seien für Y genannt:
CH₂=CH-CO-O-(CH₂)₂-; CH₂=CH-CO-NH-(CH₂)₂-; CH₂=C(CH₃)-CO-O-(CH₂)₂-; CH₂=C(CH₃)-CO-NH-(CH₂)₂-;
CH₂=CH-CO-O-(CH₂)₃-; CH₂=CH-CO-NH-(CH₂)₃-; CH₂=C(CH₃)-CO-O-(CH₂)₃-; CH₂=C(CH₃)-CO-NH-(CH₂)₃-;
CH₂=CH-CO-O-(CH₂)₄-; CH₂=CH-CO-NH-(CH₂)₄-; CH₂=C(CH₃)-CO-O-(CH₂)₄-; CH₂=C(CH₃)-CO-NH-(CH₂)₄-;

Besonders bevorzugt als Y ist CH₂=C(CH₃)-CO-O-(CH₂)₂.

R₁ und R₂, R₃, R₄,R₆, R₇, R₉ können unabhängig voneinander darstellen Wasserstoff, verzweigte oder unverzweigte C₁-C₈-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl und n-Octyl.

Als Monomer (A-2) geeignet sind Monomere der allgemeinen Formel (II)

X-C(O)CR⁷ = CHR⁶ (II),

wobei die Substituenten folgende Bedeutung besitzen:
X ausgewählt ist aus der Gruppe der Reste -OH, -OR⁸, NH₂, -NHR⁸, N(R⁸)₂;
die Reste R⁸ können identisch oder verschieden ausgewählt werden aus der Gruppe, bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl;
R⁷ und R⁶ sind unabhängig voneinander, ausgewählt aus der Gruppe, bestehend aus -H, C₁-C₈ linear- oder verzweigtkettige Alkylketten, Methoxy, Ethoxy, 2-Hydroxyethoxy, 2-Methoxyethoxy und 2-Ethoxyethyl.

Repräsentative aber nicht limitierende Beispiele von geeigneten Monomeren (A-2) sind zum Beispiel Acrylsäure und deren Ester und Amide.

Die Ester können abgeleitet sein von C₁-C₄₀ linearen, C₃-C₄₀ verzweigtkettigen, oder C₃-C₄₀ carbocyclischen Alkoholen, von mehrfachfunktionellen Alkoholen mit 2 bis etwa 8 Hydroxylgruppen wie Ethylenglycol, Hexylenglycol, Glycerin, and 1,2,6-Hexantriol, von Aminoalkoholen oder von Alkoholethern wie Methoxyethanol und Ethoxyethanol oder Polyethylenglykolen.

Ebenfalls verwendbare Monomere (A-2) sind substituierte Acrylsäuren sowie Ester und Amide davon, wobei die Substituenten an den Kohlenstoffatomen in der zwei oder drei Position der Acrylsäure stehen, und unabhängig voneinander ausgewählt sind aus der Gruppe, bestehend aus C₁-C₄ Alkyl, -CN, COOH, besonders bevorzugt Methacrylsäure, Ethacrylsäure und 3-Cyanoacrylsäure. Diese Ester und Amide dieser substituierten Acrylsäuren können wie oben für die Ester und Amide der Acrylsäure beschrieben ausgewählt werden.

Besonders geeignete Monomere (A-2) sind Acrylsäure, Methacrylsäure, Ethylacrylsäure, Methylacrylat, Ethylacrylat, Propylacrylat, n-Butylacrylat, iso-Butylacrylat, t-Butylacrylat, 2-Ethylhexylacrylat, Decylacrylat, Methylmethacrylat, Ethylmethacrylat, Propylmethacrylat, n-Butylmethacrylat, iso-Butylmethacrylat, t-Butylmethacrylat, 2-Ethylhexylmethacrylat, Decylmethacrylat, Methylethacrylat, Ethylethacrylat, n-Butylethacrylat, iso-Butylethacrylat, t-Butyl-ethacrylat, 2-Ethylhexylethacrylat, Decylethacrylat, 2,3-Dihydroxypropylacrylat, 2,3-Dihydroxypropylmethacrylat, 2-Hydroxyethylacrylat, Hydroxypropylacrylate, 2-Hydroxyethylmethacrylat, 2-Hydroxyethylethacrylat, 2-Methoxyethylacrylat, 2-Methoxyethylmethacrylat, 2-Methoxyethylethacrylat, 2-Ethoxyethylmethacrylat, 2-Ethoxyethylethacrylat, Hydroxypropylmethacrylate, Glycerylmonoacrylat, Glycerylmonomethacrylat, Polyalkylenglykol(meth)acrylate, ungesättigte Sulfonsäuren, wie z.B. Acrylamidopropansulfonsäure;

Als Monomere (A-2) geeignet sind weiterhin Maleinsäure, Fumarsäure, Maleinsäureanhydrid und seine Halbester, Crotonsäure, Itaconsäure.

Von diesen sind besonders bevorzugt Acrylsäure, Methacrylsäure, Maleinsäure, Fumarsäure, Crotonsäure, Maleinsäureanhydrid sowie dessen Halbester, Methylacrylat, Methylmethacrylat, Ethylacrylat, Ethylmethacrylat, n-Butylacrylat, n-Butylmethacrylat, t-Butylacrylat, t-Butylmethacrylat, lsobutylacrylat, Isobutylmethacrylat, 2-Ethylhexylacrylat, N-t-Butylacrylamid, N-Octylacrylamid, 2-Hydroxyethylacrylat, Hydroxypropylacrylat, 2-Hydroxyethylmethacrylat, Hydroxypropylmethacrylat, Alkylenglykol(meth)acrylate, ungesättigte Sulfonsäuren wie zum Beispiel Acrylamidopropansulfonsäure, und N-[3-(dimethylamino)propyl]-methacrylamid

Besonders geeignet als Monomere (A-2) sind n-Butylacrylat, Acrylsäure und/oder Stearylmethacrylat.

R₈ und R₁₂, R₁₃, können unabhängig voneinander darstellen verzweigte oder unverzweigte C₁₋C₄₀-Alkylreste, bevorzugt verzweigte oder unverzweigte C₁-C₂₀-Alkylreste.

Als Monomere (B-1) geeignet sind Monomere der allgemeinen Formel (III) 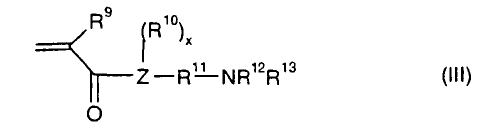 mit
- R⁹: = H, Alkyl mit 1 bis 8 C-Atomen,
- R¹⁰: = H, Methyl,
- R¹¹: = Alkylen mit 1 bis 24 C-Atomen, optional substituiert durch C₁-C₆-Alkyl,
- R¹², R¹³: = C₁-C₄₀ Alkylrest,
- Z: = Stickstoff für x = 1 oder Sauerstoff für
- x: = 0.

Die Amide können unsubstituiert, N-Alkyl oder N-alkylamino monosubstituiert, oder N,N-dialkylsubstituiert oder N,N-dialkylamino disubstituiert sein, worin die Alkyl- oder Alkylaminogruppen von C₁-C₄₀ linearen, C₃-C₄₀ verzweigtkettigen, oder C₃-C₄₀ carbocyclischen Einheiten abgeleitet sind. Zusätzlich können die Alkylaminogruppen quaternisiert werden.

Ebenfalls verwendbare Monomere (E) sind substituierte Acrylsäuren sowie, Ester und Amide davon, wobei die Substituenten an den Kohlenstoffatomen in der zwei oder drei Position der Acrylsäure stehen, und unabhängig voneinander ausgewählt sind aus der Gruppe, bestehend aus C₁-C₄ Alkyl, -CN, COOH, besonders bevorzugt Methacrylsäure, Ethacrylsäure und 3-Cyanoacrylsäure. Diese Ester und Amide dieser substituierten Acrylsäuren können wie oben für die Ester und Amide der Acrylsäure beschrieben ausgewählt werden.

Besonders bevorzugt als Monomere (B-1) werden ein oder mehrere Monomere ausgewählt aus folgender Gruppe eingesetzt: N,N-Dimethylaminomethyl(meth)acrylat, N,N-Diethylaminomethyl(meth)acrylat, N,N-Dimethylaminoethyl(meth)acrylat, N,N-Diethylaminoethyl-(meth)acrylat, N,N-Dimethylaminopropyl(meth)acrylat, N,N-Diethylaminopropyl(meth)acrylat.

In einer besonders bevorzugten Ausführungsform wird als Monomer (B-1) N,N-Dimethylaminopropyl(meth)acrylat eingesetzt.

Als geeignete copolymerisierbare Monomere (B-2) können bevorzugt ethylenisch ungesättigte Monomere verwendet werden. Dabei kann entweder ein einzelnes Monomer oder Kombinationen von zwei oder mehr Monomeren verwendet werden. Mit copolymerisierbar ist gemeint, daß die verwendeten Monomere unter Verwendung irgendeiner konventionellen synthetischen Methode polymerisiert werden können.

Beispielsweise können dies Lösungspolymerisation, Emulsionspolymerisation, umgekehrte Emulsionspolymerisation, Suspensionspolymerisation, umgekehrte Suspensionspolymerisation oder Fällungspolymerisation sein, ohne dass die verwendbaren Methoden darauf beschränkt sind. Bei der Lösungspolymerisation kann Wasser oder übliche organische Lösungsmittel als Lösungsmittel verwendet werden. Auch die Herstellung in der Schmelze ist möglich.

Monomere, die mit einer durch freie Radikale initiierten Reaktion polymerisiert werden können, sind bevorzugt. Der Begriff ethylenisch ungesättigt bedeutet, dass die Monomere zumindest eine polymerisierbare Kohlenstoff-Kohlenstoff-Doppelbindung besitzen, die mono-, di-, tri-, oder tetrasubstituiert sein kann.

Geeignet sind prinzipiell alle als (A-1) und (A-2) genannten Monomere, sofern sie von (B-1) verschieden sind.

In einer bevorzugten Ausführungsform wird als Copolymer (A) ein Copolymer eingesetzt aus mindestens einem Monomeren (A-1) und mindestens zwei Monomeren (A-2).

In einer bevorzugten Ausführungsform wird als Copolymer (A) ein Copolymer eingesetzt aus mindestens einem Monomeren (A-1) und mindestens zwei Monomeren (A-2), wobei die Monomeren (A-2) ausgewählt sind aus der Gruppe bestehend aus n-Butylacrylat, Acrylsäure und/oder Stearylmethacrylat.

In einer bevorzugten Ausführungsform wird als Copolymer (A) ein Copolymer eingesetzt, bei dem als Monomer (A-1) Ureidomethyacrylat gewählt wird und als Monomer (A-2) mindestens ein Monomer aus der Gruppe bestehend aus n-Butylacrylat, Acrylsäure und/oder Stearylmethacrylat.

In einer bevorzugten Ausführungsform der Erfindung wird als Copolymer (A) ein Copolymer eingesetzt aus mindestens einem Monomeren (A-1) und mindestens 2 Monomeren (A-2).

In einer bevorzugten Ausführungsform wird als Copolymer (A) ein Copolymer eingesetzt, bei dem als Monomer (A-1) Ureidomethyacrylat gewählt wird und als Monomer (A-2) mindestens zwei Monomere aus der Gruppe bestehend aus n-Butylacrylat, Acrylsäure und/oder Stearylmethacrylat.

In einer bevorzugten Ausführungsform wird als Copolymer (B) ein Copolymer eingesetzt aus mindestens einem Monomeren (B-1) und mindestens zwei Monomeren (B-2).

In einer bevorzugten Ausführungsform wird als Copolymer (B) ein Copolymer eingesetzt aus N,N-Dimethylaminopropyl(meth)acrylat als Monomeren (B-1) und mindestens einem weiteren Monomeren (B-2).

In einer bevorzugten Ausführungsform wird als Copolymer (B) ein Copolymer eingesetzt aus N,N-Dimethylaminopropyl(meth)acrylat als Monomeren (B-1) und mindestens einem weiteren Monomeren (B-2) ausgewählt aus der Gruppe bestehend aus n-Butylacrylat und Ureidomethacrylat.

In einer bevorzugten Ausführungsform werden die molaren Verhältnisse der Copolymere (A) zu (B) so gewählt, dass sie im Bereich von 1 :10 bis 10 :1, insbesonderen im Bereich von 1 : 5 bis 5 : 1 liegen.

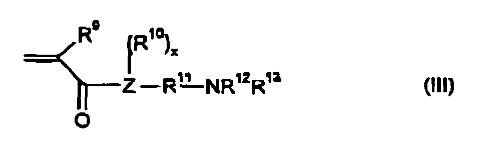

Die erfindungsgemäßen Zubereitungen eignen sich als Verdicker. Ein weiterer Gegenstand der vorliegenden Erfindung betrifft ein Verfahren zur Verdickung von kosmetischen Zubereitungen, bei dem man der zu verdickenden Zubereitung 1 bis 30 Gew.-%, insbesondere 5 bis 25 Gew.-%, bevorzugt 8 bis 20 Gew.-%, einer Zusammensetzung nach Anspruch 1 zugibt.

Die erfindungsgemäßen Zubereitungen können vorteilhaft in kosmetischen Zubereitungen verwendet werden, insbesondere haarkosmetischen Zubereitungen.

Der Begriff der kosmetischen Zubereitungen ist breit zu verstehen und meint all solche Zubereitungen, die sich zum Auftragen auf Haut und/oder Haare und/oder Nagel eignen und einen anderen als einen ausschließlich medizinisch-therapeutischen Zweck verfolgen.

Die erfindungsgemäßen Zubereitungen können in hautkosmetischen Zubereitungen eingesetzt werden.

Beispielsweise werden die erfindungsgemäßen Zubereitungen kosmetischen Mitteln zur Reinigung der Haut verwendet Solche kosmetischen Reinigungsmittel sind ausgewählt aus Stückseifen, wie Toilettenseifen, Kernseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch- und Badepraparaten, wie Waschlotionen, Duschbädern und -gelen, Schaumbädern, Ölbädern und Scrub-Präparaten.

Bevorzugt werden die erfindungsgemäßen Zubereitungen in kosmetischen Mitteln zur Pflage und zum Schutz der Haut, in Nagelpflegemitteln sowie in Zubereitungen für die dekorative Kosmetik angewendet

Besonders bevorzugt ist die Verwendung in Hautpflegemitteln, Intimpflegemitteln, Fußpflegemitteln, Deodorantien, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudem und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen Zubereitungen können die erfindungsgemäßen Zubereitungen besondere Wirkungen entfalten. Die Zubereitungen können unter anderem zur Feuchthaltung und Konditionierung der Haut und zur Verbesserung des Hautgefühls beitragen. Die Zubereitungen können auch als Verdicker in den Formulierungen wirken. Durch Zusatz der erfindungsgemäßen Zubereitungen kann in bestimmten Formulierungen eine erhebliche Verbesserung der Hautverträglichkeit erreicht werden.

Die Copolymere sind in den hautkosmetischen Zubereitungen in einem Anteil von etwa 0.001 bis 20 Gew.-%, vorzugsweise 0,01 bis 10 Gew.-%, ganz besonders bevorzugt 0,1 bis 5 Gew.-%, bezogen auf das Gesamtgewicht des Mittels, enthalten.

Je nach Anwendungsgebiet können die erfindungsgemäßen Mittel in einer zur Hautpflege geeigneten Form, wie z.B. als Creme, Schaum, Gel, Stift. Pulver, Mousse, Milch oder Lotion appliziert werden.

Die hautkosmetischen Zubereitungen können neben den erfindungsgemäßen Zubereitungen und geeigneten Lösungsmitteln noch in der Kosmetik übliche Zusätze, wie Emulgatoren, Konservierungsmittel, Parfümöle, kosmetische Wirkstoffe wie Phytantriol, Vitamin A, E und C, Retinol, Bisabolol. Panthenol, Lichtschutzmittel, Bleichmittel, Färbemittel, Tönungsmittel, Bräunungsmittel (z.B. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als geeignete Lösungsmittel sind insbesondere zu nennen Wasser und niedrige Monoalkohole oder Polyole mit 1 bis 6 Kohlenstoffatomen oder Mischungen davon: bevorzugte Monoalkohole oder Polyole sind Ethanol, i-Propanol, Propylenglycol, Glycerin und Sorbit.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoff-atomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀₋Fettsäuren, Wachsester, wie z.B. Jojobaöl, Fettalkohole, Vaseline, hydriertes Lanolin und azetyliertes Lanolin. Selbstverständlich können auch Mischungen derselben verwendet werden.

Übliche VerdicKungsmittel in derartigen Formulierungen sind vernetzte Polyacrylsäuren und deren Derivate, Polysaccharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Man kann die erfindungsgemäßen Zubereitungen auch mit herkömmlichen Polymeren abmischen, falls spezielle Eigenschaften eingestellt werden sollen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer™ 100P), Copolymere aus Ethylacrylat und Methacrylsäure (z.B. Luvimer™ MAE), Copolymere aus N-tert.-Butyl-acrylamid, Ethylacrylat, Acrylsäure (Ultrahold™ 8, strong), Copolymere aus Vinylacetat, Crotonsäure und gegebenenfalls weitere Vinylester (z.B. Luviset™ Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol™ VBM), Copolymere von Acrylsäure und Methacrylsäure mit hydrophoben Monomeren, wie z.B. C₄-C₃₀-Alkylester der Meth(acrylsäure), C₄₋C₃₀-Alkylvinylester, C₄-C₃₀-Alkylvinylether und Hyaluronsäure.

Weitere geeignete Polymere sind kationische Polymere mit der Bezeichnung Polyquaternium nach INCI, z.B. Copolymere aus Vinylpyrrolidon/N-Vinylimidazoliumsalzen (Luviquat™ FC, Luviquat™ HM, Luviquat™ MS, Luviquat™ Care), Copolymere aus N-Vinylpyrrolidon/Dimethylaminoethylmethacrylat, quaternisiert mit Diethylsulfat (Luviquat™ PQ 11), Copolymere aus N-Vinylcaprolactam/N-Vinylpyrrolidon/N-Vinylimidazoliumsalzen (LuviquatTM Hold); kationische Cellulosederivate (Polyquaternium-4 und -10), Acrylamidcopolymere (Polyquaternium-7) und Chitosan.

Als weitere Polymere sind auch neutrale Polymere geeignet wie Polyvinylpyrrolidone, Copolymere aus N-Vinylpyrrolidon und Vinyl-acetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Die Copolymerisate werden in kosmetischen Zubereitungen eingesetzt, deren Herstellung nach den üblichen dem Fachmann geläufigen Regeln erfolgt

Solche Formulierungen liegen vorteilhafterweise in Form von Emulsionen bevorzugt als Wasser-in-Öl-(W/O)- oder Öl-in-Wasser-(O/W)-Emulsionen vor. Es Ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W- oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben den Copolymerisaten übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage. 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wässrige Phase, die mittels eines geeigneten Emulgatorsystems in einer Öl- oder Fettphase emulgiert ist

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wässrige Phasen etwa 20 und 70 Gew.-%, jeweils bezogen auf das Gesamtgewicht der Emulsion. Bei den Emulgatoren handelt es sich um diejenigen, welche in diesem Emulsionstyp üblicherweise verwendet werden. Sie werden z.B. ausgewählt unter. C₁₂-C₁₈-SorbitanFettsäureestern; Estern von Hydroxystearinsäure und C₁₂-C₂₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolin-alkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Silikonglycol-Copolymer, Fettsäuren und Fettalkohole enthalten.

Um die Retention von Ölen zu begünstigen, kann man auch Wachse verwenden, wie z.B. Carnauba-Wachs, Candellilawachs, Bienenwachs, mikrokristallines Wachs, Ozokeritwachs und Ca, Mg- und Al-Oleate, -Myristate, -Linoleate und -Stearate.

Im allgemeinen werden diese Wasser-in-Öl-Emulsionen so hergestellt, dass die Fettphase und der Emulgator in den Ansatzbehälter gegeben werden. Man erwärmt diesen bei einer Temperatur von 70 bis 75°C, gibt dann die in Öl löslichen Ingredienzen zu und fügt unter Rühren Wasser hinzu, welches vorher auf die gleiche Temperatur erwärmt wurde und worin man die wasserlöslichen Ingredienzen vorher gelöst hat; man rührt, bis man eine Emulsion der gewünschten Feinheit hat, lässt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt.

Die wässrige Phase der O/W-Emulsion der erfindungsgemäßen Zubereitungen enthält gegebenenfalls
- Alkohole, Diole oder Polyole sowie deren Ether, vorzugsweise Ethanol, Isopropanol, Propylenglycol, Glycerin, Ethylenglycolmonoethylether;
- übliche Verdickungsmittel bzw. Gelbildner, wie z.B. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:
- Ester aus gesättigten und/oder ungesättigten, verzweigten und/oder unverzweigten C₃₋C₃₀-Alkancarbonsäuren und gesättigten und/oder ungesättigten, verzweigten und/oder unverzweigten C₃-C₃₀-Alkoholen, aus aromatischen Carbonsäuren und gesättigten und/oder ungesättigten, verzweigten und/oder unverzweigten C₃-C₃₀-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter C₈₋C₂₄-Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Die erfindungsgemäßen Zubereitungen eignen sich auch zur Verwendung in Wasch- und Duschgel-Formulierungen sowie Badepräparaten.

Solche Formulierungen enthalten neben den erfindungsgemäßen Zubereitungen üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäure-ester von Polyethylenglykolen, ethoxylierte Fettsäureamide, Alkylpolyglykoside oder Sorbitanetherester geeignet.

Außerdem können die Wasch, Dusch- und Badepräparate übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-4, -10), Guar-hydroxypropyltrimethylammoniumchlorid (INCI: Hydroxypropyl Guar Hydroxypropyltrimonium Chloride), Copolymere aus N-Vinylpyrrolidon und quaternisiertem N-Vinylimidazol (Polyquaternium-16, -44, -46), Copolymere aus N-Vinypyrrolidon/Dimethylaminoethyl-methacrylat, quaternisiert mit Diethylsulfat (Polyquaternium-11) und andere.

Weiterhin können die Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

Haarkosmetische Zubereitungen umfassen insbesondere Stylingmittel und/oder Konditioniermittel in haarkosmetischen Zubereitungen wie Haarkuren, Haarschäume (engl. Mousses), (Haar)gelen oder Haarsprays, Haarlotionen, Haarspülungen, Haarshampoos, Haaremulsionen, Spitzenfluids, Egalisierungsmittel für Dauerwellen, Haarfärbe- und -bleichmittel, "Hot-Oil-Treatment"- Präparate, Conditioner, Festigerlotionen oder Haarsprays. Je nach Anwendungsgebiet können die haarkosmetischen Zubereitungen als (Aerosol-)Spray, (Aerosol-)Schaum, Gel, Gelspray, Creme, Lotion oder Wachs appliziert werden.

Die erfindungsgemäßen haarkosmetischen Formulierungen enthalten in einer bevorzugten Ausführungsform
a) 0,05 bis 20 Gew.-% der erfindungsgemäßen Zubereitung
b) 20 bis 99,95 Gew.-% Wasser und/oder Alkohol
c) 0 bis 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Trübungsmittel, Wirkstoffe, UV-Filter, Pflegestoffe wie Panthenol, Collagen. Vitamine, Eiweißhydrolysate, Alpha- und Beta-Hydroxycarbonsäuren, Eiweißhydrolysate, Stabilisatoren. pH-Wert-Regulatoren, Farbstoffe, Viskositätsregullerer, Gelbildner, Farbstoffe, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze: Natriumsalze von Polyhydroxycarbonsäuren, wasserlösliche oder wasserdispergierbare Polyester, Polyurethane (Luviset™ P.U.R.) und Polyharnstoffe. Besonders geeignete Polymere sind Copolymere aus t-Butylacrylat, Ethylacrylat, Methacrylsäure (z.B. Luvimer™ 100P), Copolymere aus N-tert.-Butylacrylamid, Ethylacrylat, Acrylsäure (Ultrahold™ 8, Strong), Copolymere aus Vinylacetat, Crotonsäure und gegebenenfalls weiteren Vinylestern (z.B. Luviset™ Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol™ VBM).

Weiterhin umfasst die Gruppe der zur Kombination mit den Polymerisaten geeigneten Polymere beispielhaft Balance®+ CR (National Starch; Acrylatcopolymer), Balance®+ 0/55 (National Starch; Acrylatcopolymer), Balance®+ 47 (National Starch; Octylacrylamid/Acrylat/Butylaminoethylmethacrylate-Copolymer), Aquaflex® FX 64 (ISP; Isobutylen/Ethylmaleimid/Hydroxyethylmaleimid-Copolymer), Aquaflex® SF-40 (ISP / National Starch; VP/Vinyl Caprolactam/DMAPA Acrylatcopolymer), Allianz® LT-120 (ISP / Rohm & Haas; Acrylat/C1-2 Succinat/Hydroxyacrylat-Copolymer). Aquarez® HS (Eastman; Polyester-1). Diaformer® Z-400 (Clariant; Methacryloylethylbetain/Methacrylat-Copolymer), Diaformer® Z-711 (Clariant; Methacryloylethyl N-oxid/Methacrylat-Copolymer), Diaformer® Z-712 (Clariant; Methacryloylethyl N-oxide/Methacrylat-Copolymer), Omnirez® 2000 (ISP⁻; Monoethylester von Poly(Methylvinylether/Maleinsäure in Ethanol), Amphomer® HC (National Starch; Acrylat/ Octylacrylamid-Copolymer), Amphomer® 28-4910 (National Starch; Octylacrylamid/Acrylat/Butylaminoethylmethacrylat-Copolymer), Advantage® HC 37 (ISP; Terpolymer aus Vinylcaprolactam/Vinylpyrrolidon/Dimethylaminoethylmethacrylat), Acudyne®+ 258 (Rohm & Haas; Acrylat/ Hydroxyesteracrylat-Copolymer), Luviset® PUR (BASF, Polyurethane-1), Luviflex® Silk (BASF), Eastman® AQ48 (Eastman).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCI, z.B. Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam N-Vinylpyrrolidon/N-Vinylimidazoliumsalzen (Luviquat® Hold); kationische Cellulosederivate (Polyquaternium-4 und -10), Acrylamidcopolymere (Polyquaternium-7).

Ferner können kationische Guarderivate wie Guarhydroxypropyltrimoniumchlorid (INCI) verwendet werden.

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze oder Dimethicon Copolyole (CTFA) und aminofunktionelle Silikonverbindungen wie Amodimethicone (CTFA).

Die erfindungsgemäßen Zubereitungen eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten diese Zubereitungen
a) 0,1 bis 10 Gew.-% der erfindungsgemäßen Zubereitung
b) 20 bis 99,9 Gew.-% Wasser und/oder Alkohol
c) 0 bis 70 Gew.-% eines Treibmittel
d) 0 bis 20 Gew.-% weitere Bestandteile

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.

Eine erfindungsgemäß bevorzugte Formulierung für Aerosolhaarschäume enthält
a) 0,1 bis 10 Gew.-% der erfindungsgemäßen Zubereitung
b) 55 bis 99,8 Gew.-% Wasser und/oder Alkohol
c) 5 bis 20 Gew.-% eines Treibmittel
d) 0,1 bis 5 Gew.-% eines Emulgators
e) 0 bis 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCI-Nomenklatur) sind Laurethe, z.B. Laureth-4; Cetethe, z.B. Cetheth-1, Polyethylenglycolcetylether; Ceteareth, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyldimethyl-2-hydroxyethylammoniumdihydrogenphosphat, Cetyltrimoniumchlorid, Cetyltrimmoniumbromid, Cocotrimoniummethylsulfat, Quaternium-1 bis x (INCl).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Eine erfindungsgemäß für Styling-Gele geeignete Zubereitung kann beispielsweise wie folgt zusammengesetzt sein:
a) 0,1 bis 10 Gew.-% der erfindungsgemäßen Zubereitung
b) 60 bis 99,85 Gew.-% Wasser und/oder Alkohol
c) 0,05 bis 10 Gew.-% eines Gelbildners
d) 0 bis 20 Gew.-% weitere Bestandteile

Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer. Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquaternium-7, Polyquaternium-44.

Die erfindungsgemäßen Zubereitungen erhöhen die Kämmbarkeit von Haaren, sie können daher als Konditioniermitteln, insbesondere als Konditioniermittel in kosmetischen Zubereitungen eingesetzt werden

Die erfindungsgemäßen Zubereitungen können in kosmetischen Zubereitungen als Verdicker eingesetzt werden.

Bevorzugte Shampooformulierungen enthalten
a) 0,05 bis 10 Gew.-% der erfindungsgemäßen Zubereitungen
b) 25 bis 94,95 Gew.-% Wasser
c) 5 - 50 Gew.-% Tenside
c) 0 - 5 Gew.-% eines weiteren Konditioniermittels
d) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäureester von Polyethylenglykolen. Alkylpolyglykoside oder Sorbitanetherester geeignet

Außerdem können die Shampooformulierungen übliche kationische Tenside enthalten, wie z.B. quatemäre Ammoniumverbinclungen, beispielsweise Cetyltrimethylammoniumchlorid.

In den Shampooformulierungen können zur Erzielung bestimmter Effekte übliche Konditioniermittel in Kombination mit den Polymerisaten eingesetzt werden. Hierzu zählen beispielsweise kationische Polymere mit der Bezeichnung Polyquaternium nach INCI, insbesondere Copolymere aus Vinylpyrrolidon/N-Vinylimidazoliumsalzen (Luviquat® FC, Luviquat® HM, Luviquat® MS, Luviquat® Care), Copolymere aus N-Vinylpyrrolidon/Dimethylaminoethylmethacrylat, quaternisiert mit Diethylsulfat (Luviquat® PQ 11), Copolymere aus N-Vinylcaprolactam/N-Vinylpyrrolidon/N-Vinylimidazoliumsalzen (Luviquat® Hold); kationische Cellulosederivate (Polyquaternium-4 und -10), Acrylamidcopolymere (Polyquaternium-7). Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze, Weitere geeignete Silikonverbindungen sind Dimethicon Copolyole (CTFA) und aminofunktionelle Silikonverbindungen wie Amodimethicone (CTFA). Ferner können kationische Guarderivate wie Guarhydroxypropyltrimoniumchlorid (INCI) verwendet werden.

### Herstellungsbeispiele

### Alle Copolymere (A) bzw. (B) wurden via Lösungspolymerisation wie folgt hergestellt:

Eine Lösung der verschiedenen Monomere (10 % der Gesamtmenge, die eingesetzt wird) und eine Lösung enthaltend 1 % WAKO V 59 in Ethanol (13 % der Gesamtmenge, die eingesetzt wird) in Ethanol (70 bis 250 g, je nach Polymer) wurde unter Stickstoffatmosphäre auf 80°C erhitzt. Zwei verschieden Lösungen wurden tropfenweise zu dieser Lösung gegeben: 1) Eine Lösung mit dem Initiator [WAKO V59 0.99 g in Ethanol (100 g)]; 2) eine Lösung mit den gewählten Monomeren in den ausgewählten prozentualen Mengen. Die initiatorlösung 1) wurde über einen Zeitraum von 4 h zugetropft, die Monomerenlösung 2) über einen Zeitraum von 3 h. Die Reaktionstemperatur wurde bei 80°C gehalten und die Lösungen wurden bei ständigem Rühren unter Stickstoffatmosphäre gehalten. Eine Stunde nach der letzten Zugabe wurde unter Beibehalt der Reaktionsbedingungen eine Lösung von 2,31 g WAKO V59 in 100 g Ethanol über einen Zeitraum von 1 h zugegeben. Die erhaltende Reaktionsmischung wurde weitere 4 h bei 80°C belassen (unter ständigem Rühren und Stickstoff Atmosphäre). Tabelle 1 gibt die verschiedenen Monomeren sowie die Mol-% der Monomere im entsprechenden Copolymer wieder.

### Viskositätsmessung

Die Viskosität wurde für jedes Polymer wurde in einem Wells-Brookfield-CONE/PLATE-Viskosimeter mit Cone CP-41 (3°) bei 23°C gestimmt. Dazu wurden ca. 2 ml der Probe in den temperierten Messkörper gegeben (Platte war während der Messing komplett benetzt). Die Viskositäten wurden in mPa·s bestimmt

Die Viskositäten wurden dabei jeweils unter 3 verschiedenen Bedingungen gemessen:
I) in einer ethanolischen Lösung mit 40 % Feststoffanteil
II) in einer 50:50 Mischung aus a) und Miglyol
III) in einer 20:80 Mischung aus a) und Miglyol (= 80)

Die entsprechenden Ergebnisse finden sich in Tabelle 1.

**Tabelle 1: Synthetisierte Copolymere (A) und (B) und ihre Viskositäten**

| *Nr.* | Monomeren Zusammensetzung (Mol-%) | Viskosität I (mPas) | Viskosität II (mPas) | Viskosität III (mPas) | Lösung III |
|---|---|---|---|---|---|
| | ⁿBA:DMAPMA (Copolymer B) | | | | |
| 5 | 95:5 | 37 | 31 | 25 | Klar |
| 6 | 90:10 | 46 | 39 | 28 | Klar |
| 7 | 80:20 | 62 | 59 | 38 | Klar |
| 8 | 70:30 | 74 | 47 | 33 | Klar |

| | ⁿBA: DMAPMA (Copolymer B) | | | | |
|---|---|---|---|---|---|
| 9 | 88:12 | 80 | 59 | 38 | Klar |

| | ⁿBA:UMA:AS (Copolymer A) | | | | |
|---|---|---|---|---|---|
| 10 | 80:10:10 | 122 | 105 | N. m. ^{(a)} | Ph. ^{(b)} |
| 11 | 70:10:20 | 82 | 55 | N. m. ^{(a)} | Ph. ^{(b)} |
| 12 | 60:10:30 | 237 | 138 | N. m. ^{(a)} | Ph. ^{(b)} |

| | ⁿBA:UMA:DMAPMA (Copolymer B) | | | | |
|---|---|---|---|---|---|
| 13 | 80:10:10 | 52 | 44 | 29 | Klar |
| 14 | 70:10:20 | 64 | 50 | 32 | Klar |
| 15 | 60:10:30 | 144 | 94 | 44 | Klar |
| | SMA:ⁿBA:UMA:AS (Copolymer A) | | | | |

| | | | | | |
|---|---|---|---|---|---|
| ^{(a)}: nicht mischbar ^{(b)}: Phasentrennung | | | | | |

Die Viskosität von Miglyol alleine beträgt 22 mPas
Alle Copolymere der Tabelle I ließen sich in Lösungen I und II (I = 40 gew.-%ige ethanolische bzw. II = 50:50 Lösung aus I und Miglyol) als klare Lösung herstellen.

Miglyol 812 ist unter dem INCI Namen Caprylic/Capric Triglyceride bekannt.

Herstellung der erfindungsgemäßen Mischungen aus Copolymeren (A) und Copolymeren (B)

Die entsprechenden Copolymeren (A) und (B) wurden als Mischungen bei Raumtemperatur in verschiedenen Gewichtsverhältnissen wie im folgenden zueinander hergestellt. Folgende Mischungen wurden hergestellt (Gewichtsverhältnisse der Copolymeren (A) zu (B) sind in Tabelle 2 angegeben.

### Verwendete Abkürzungen

- nBA: n-Butylacrylat
- AS: Acrylsäure
- DMAPMA: N,N-Dimethylaminopropyl(meth)acrylat
- SMA: Stearylmethacrylat
- UMA: Ureidomethacrylat

| Mischung | Copolymer (A) | Copolymer (B) |
|---|---|---|
| (1) | ⁿBA:UMA:AS | ⁿBA: DMAPMA |
| (2) | ⁿBA:UMA:AS | ⁿBA:UMA:DMAPMA |
| (3) | SMA:ⁿBA:UMA:AS | ⁿBA:UMA:DMAPMA |

Aus den Copolymeren (A) und (B) wurden verschiedene Mischungen hergestellt. Diese wurden in unterschiedlichen Gewichtsverhältnissen mit Miglyol gemischt und die Viskosität wie oben angegeben gemessen. Tabelle 2 stellt die Ergebnisse zusammen.

**Tabelle 2: Mischungen aus Copolymer (A) ⁿBA:UMA:AS und Copolymer (B) ⁿBA:DMAPMA**

| Nr. | (A) ⁿBA : UMA : AS (%Mol) | (B) ⁿBA : DMAPMA (%Mol) | (A) : (B) Gew.-Ratio | [(A)-(B)] : Miglyol Gew.-Ratio | Viskosität (mPas) |
|---|---|---|---|---|---|
| 19a | 80:10:10 | 90:10 | 1:0,7 | - | 2015 |
| 19b | | | | 50:50 | 1350 |
| 19c | | | | 20:80 | N.m. ^{(a)} |
| 19d | | | 1 : 1,02 | - | 2655 |
| 19e | | | | 50:50 | 1573 |
| 19f | | | | 20:80 | N.m.^{(a)} |
| 20a | 70:10:20 | 80:20 | 1:0,7 | - | - |
| 20b | | | | 50:50 | N.m. ^{(a)} |
| 20c | | | | 20:80 | N.m. ^{(a)} |
| 20d | | | 1 : 1,10 | - | 10076 |
| 20e | | | | 50:50 | N.m. ^{(a)} |
| 20f | | | | 20:80 | N.m. ^{(a)} |
| 21 a | 60 : 10 : 30 | 1 : 0,7 | - | | H. v. ^{(b)} |
| 21 b | | | 50:50 | | N.m. ^{(a)} |
| 21c | | | 20:80 | | N.m. ^{(a)} |
| 21d | | | 1 : 1,19 | - | H. v. ^{(b)} |
| 21 e | | | | 50:50 | N.m. ^{(a)} |
| 21f | | | | 20:80 | N.m. ^{(a)} |
| 22a | 80:10:10 | 88:12 | 1:1 | - | 1740 |
| 22b | | | | 50:50 | 832 |
| 22c | | | | 40:60 | 750 |
| 22d | | | | 30:70 | N.m. ^{(a)} |
| 22e | | | | 20:80 | N.m. ^{(a)} |
| 23a | 70:10:20 | 88:12 | 1:2 | - | 2310 |
| 23b | | | | 50:50 | 909 |
| 23c | | | | 40:60 | 639 |
| 23d | | | | 30:70 | N.m. ^{(a)} |
| 23e | | | | 20:80 | N.m. ^{(a)} |
| 24a | 60:10:30 | 88:12 | 1:3 | - | 3640 |
| 24b | | | | 50:50 | 2700 |
| 24c | | | | 40:60 | 1819 |
| 24d | | | | 30:70 | N.m. ^{(a)} |
| 24e | | | | 20:80 | N.m. ^{(a)} |

| | | | | | |
|---|---|---|---|---|---|
| (a) n.m. nicht gemessen (b) H.v. hoch viskos | | | | | |

## Patentansprüche

1. Zusammensetzung, enthaltend
(A) Copolymer aus
(A-1) mindestens einem ethylenisch ungesättigten, radikalisch copolymerisierbaren Monomeren der allgemeinen Formel (I)
Y-NR¹-C(V)-NHR² (I),
wobei die Substituenten folgende Bedeutung besitzen:
Y = einem zur radikalischen Polymerisation befähigten ethylenisch ungesättigten Rest
V = O, S oder NH
R¹, R² = unabhängig voneinander H oder eine C₁- bis C₈-Alkylgruppe, oder beide zusammen eine überbrückende C₂- bis C₄-Alkylengruppe, die bis zu zweifach mit einer C₁- bis C₄-Alkoxygruppe und/oder Hydroxylgruppe substituiert sein kann,
(A-2) mindestens einem ungesättigten Monomeren der allgemeinen Formel (II)
X-C(O)CR⁷ = CHR⁶ (II),
wobei die Substituenten folgende Bedeutung besitzen:
X ausgewählt ist aus der Gruppe der Reste -OH, -OR⁸, NH₂, -NHR⁸, N(R⁸)₂;
die Reste R⁸ können identisch oder verschieden ausgewählt werden aus der Gruppe, bestehend aus -H, C₁-C₄₀ linear- oder verzweigtkettige Alkylreste, N,N-Dimethylaminoethyl, 2-Hydroxyethyl, 2-Methoxyethyl, 2-Ethoxyethyl, Hydroxypropyl, Methoxypropyl oder Ethoxypropyl;
R⁷ und R⁶ sind unabhängig voneinander, ausgewählt aus der Gruppe, bestehend aus -H, C₁-C₈ linear- oder verzweigtkettige Alkylketten, Methoxy, Ethoxy, 2-Hydroxyethoxy, 2-Methoxyethoxy und 2-Ethoxyethyl.
(B) mindestens einem weiteren von (A) verschiedenem Copolymeren aus
(B-1) mindestens einem Monomeren der allgemeinen Formel (III) 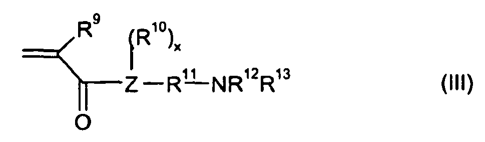 mit
R⁹ = H, Alkyl mit 1 bis 8 C-Atomen,
R¹⁰ = H, Methyl,
R¹¹ = Alkylen mit 1 bis 24 C-Atomen, optional substituiert durch C₁-C₆-Alkyl,
R¹², R¹³ = C₁-C₄₀ Alkylrest,
Z = Stickstoff für x = 1 oder Sauerstoff für
x=0.
und
(B-2) mindestens einem ethylenisch ungesättigten Monomeren.

2. Zusammensetzung nach Anspruch 1, **dadurch gekennzeichnet, dass** als Copolymer (A) ein Copolymer aus mindestens einem Monomeren (A-1) und mindestens zwei Monomeren (A-2) eingesetzt wird.

3. Zusammensetzung nach Anspruch 1, **dadurch gekennzeichnet, dass** als Monomer (A-1) eine Verbindung der folgende Formel (la) eingesetzt wird 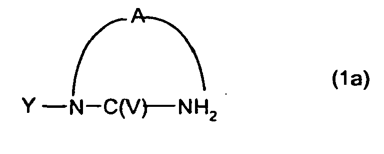 wobei A = eine 2 oder 3 gliedrige, gegebenenfalls eine Carbonylgruppe aufweisende Alkylengruppe darstellt.

4. Zusammensetzung nach Anspruch 1, **dadurch gekennzeichnet, dass** als Monomer (A-1) eine Verbindung der Formel (Ib) eingesetzt wird
wobei R³ und R⁴ unabhängig voneinander für H, -OH, -NH, C₁- bis C₈-Alkyl stehen.

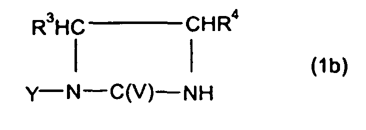

5. Zusammensetzung nach Anspruch 4, wobei R³ und R⁴ = H.

6. Zusammensetzung nach einem der vorgenannten Ansprüche, **dadurch gekennzeichnet, dass** als Monomer (A-1) eine Verbindung eingesetzt wird in der V = O.

7. Zusammensetzung nach einem der vorgenannten Ansprüche, **dadurch gekennzeichnet, dass** als Monomer (A-1) eine Verbindung der Formel (Ib) mit R³ und R⁴ = H und V = O, und Y = CH₂=C(CH₃)-CO-O-(CH₂)₂- eingesetzt wird.

8. Zusammensetzung nach einem der vorgenannten Ansprüche, **dadurch gekennzeichnet, dass** der zur radikalischen Polymerisation befähigte, ethylenisch ungesättigte Rest Y ein Rest der Formel (IV) darstellt
Y = CH₂=CR⁵-CO-W-(CH₂)ₙ- (IV),
wobei
R⁵ = H, CH₃
W = O, NH
n = 2 bis 8, insbesondere 2 bis 4
darstellt.

9. Zusammensetzung nach einem der vorgenannten Ansprüche, **dadurch gekennzeichnet, dass** als Monomer (B-1) mindestens ein Monomer eingesetzt wird, das ausgewählt ist aus der Gruppe bestehend aus N,N-Dimethylaminomethyl(meth)acrylat, N,N-Diethylaminomethyl(meth)acrylat, N,N-Dimethylaminoethyl(meth)acrylat, N,N-Diethylaminoethyl-(meth)acrylat, N,N-Dimethylaminopropyl(meth)acrylat, N,N-Diethylaminopropyl(meth)acrylat.

10. Zusammensetzung nach einem der vorgenannten Ansprüche, **dadurch gekennzeichnet, dass** als Monomer (B-1) N,N-Dimethylaminopropyl(meth)acrylat eingesetzt wird.

11. Zusammensetzung nach einem der vorgenannten Ansprüche, **dadurch gekennzeichnet, dass** als Copolymer (A) ein Copolymer eingesetzt wird aus (A-1) Ureidomethacrylat und (A-2) mindestens 2 weiteren Monomeren ausgewählt aus der Gruppe bestehend aus n-Butylacrylat, Acrylsäure und Stearylmethacrylat.

12. Zusammensetzung nach einem der vorgenannten Ansprüche, **dadurch gekennzeichnet, dass** als Copolymer (B) ein Copolymer eingesetzt wird aus (B-1) N,N-Dimethylaminopropyl(meth)acrylat und mindestens einem weiteren Monomeren ausgewählt aus der Gruppe bestehend aus n-Butylacrylat und Ureidomethacrylat.

13. Zusammensetzung nach einem der vorgenannten Ansprüche, **dadurch gekennzeichnet, dass** das (molare) Verhältnis von Copolymer (A) zu Copolymer (B) im Bereich von 1:10 bis 10:1, insbesondere im Bereich von 1:5 bis 5:1 liegt.

14. Verwendung einer Zusammensetzung nach einem der vorgenannten Ansprüche als Verdicker.

15. Verwendung einer Zusammensetzung nach einem der vorgenannten Ansprüche als Konditioniermittel.

16. Verwendung einer Zusammensetzung nach einem der vorgenannten Ansprüche in kosmetischen Zubereitungen.

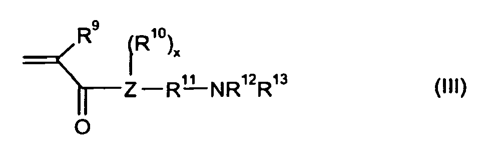

17. Verfahren zur Verdickung von kosmetischen Zubereitungen, bei dem man der zu verdickenden Zubereitung 1 bis 30 Gew.-%, insbesondere 5 bis 25 Gew.-%. bevorzugt 8 bis 20 Gew.%, einer Zusammensetzung nach Anspruch 1 zugibt.

## Claims

1. A composition comprising
(A) copolymer of
(A-1) at least one ethylenically unsaturated, free-radically copolymerizable monomer of the formula (I)
Y-NR¹-C(V)-NHR² (I),
where the substituents have the following meanings:
Y = an ethylenically unsaturated radical capable of free-radical polymerization
V = O, S or NH
R¹, R² = independently of one another H or a C₁-C₈-alkyl group, or both together a bridging C₂-C₄-alkylene group which may be substituted up to twice by a C₁-C₄-alkoxy group and/or hydroxyl group,
(A-2) at least one unsaturated monomer of the formula (II)
X-C(O)CR⁷ = CHR⁶ (II),
where the substituents have the following meanings:
X is chosen from the group of radicals -OH, -OR⁸, NH₂, -NHR⁸, N(R⁸)₂;
the radicals R⁸ may be identical or different and are chosen from the group consisting of -H, C₁-C₄₀ linear- or branched-chain alkyl radicals, N,N-dimethylaminoethyl, 2-hydroxyethyl, 2-methoxyethyl, 2-ethoxyethyl, hydroxypropyl, methoxypropyl or ethoxypropyl;
R⁷ and R⁶ are independently of one another chosen from the group consisting of -H, C₁-C₈ linear- or branched-chain alkyl chains, methoxy, ethoxy, 2-hydroxyethoxy, 2-methoxyethoxy and 2-ethoxyethyl,
(B) at least one further copolymer different from (A) of
(B-1) at least one monomer of the formula (III) 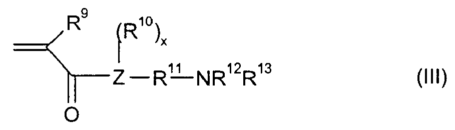 where
R⁹ = H, alkyl having 1 to 8 carbon atoms,
R¹⁰ = H, methyl,
R¹¹ = alkylene having 1 to 24 carbon atoms, optionally substituted by C₁-C₆-alkyl,
R¹², R¹³ = C₁-C₄₀-alkyl radical,
Z = nitrogen when x = 1 or oxygen when x = 0.
and
(B-2) at least one ethylenically unsaturated monomer.

2. The composition according to claim 1, wherein a copolymer of at least one monomer (A-1) and at least two monomers (A-2) is used as copolymer (A).

3. The composition according to claim 1, wherein a compound of the following formula (Ia) is used as monomer (A-1) 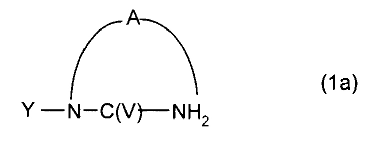 where A = a 2- or 3-membered alkylene group optionally having a carbonyl group.

4. The composition according to claim 1, wherein a compound of the formula (Ib) is used as monomer (A-1)
where R³ and R⁴, independently of one another, are H, -OH, -NH, C₁-C₈-alkyl.

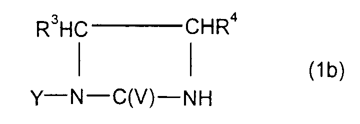

5. The composition according to claim 4, where R³ and R⁴ = H.

6. The composition according to any of the preceding claims, wherein a compound in which V = O is used as monomer (A-1).

7. The composition according to any of the preceding claims, wherein a compound of the formula (lb) where R³ and R⁴ = H and V = O, and Y = CH₂=C(CH₃)-CO-O-(CH₂)₂- is used as monomer (A-1).

8. The composition according to any of the preceding claims, wherein the ethylenically unsaturated radical Y capable of free-radical polymerization is a radical of the formula (IV)
Y = CH₂=CR⁵-CO-W-(CH₂)ₙ- (IV),
where
R⁵ = H, CH₃
W = O, NH
n = 2 to 8, in particular 2 to 4.

9. The composition according to any of the preceding claims, wherein at least one monomer which is chosen from the group consisting of N,N-dimethylaminomethyl (meth)acrylate, N,N-diethylaminomethyl (meth)acrylate, N,N-dimethylaminoethyl (meth)acrylate, N,N-diethylaminoethyl (meth)acrylate, N,N-dimethylaminopropyl (meth)acrylate, N,N-diethylaminopropyl (meth)acrylate is used as monomer (B-1).

10. The composition according to any of the preceding claims, wherein N,N-dimethylaminopropyl (meth)acrylate is used as monomer (B-1).

11. The composition according to any of the preceding claims, wherein a copolymer of (A-1) ureidomethacrylate and (A-2) at least 2 further monomers chosen from the group consisting of n-butylacrylate, acrylic acid and stearyl methacrylate is used as copolymer (A).

12. The composition according to any of the preceding claims, wherein a copolymer of (B-1) N,N-dimethylaminopropyl (meth)acrylate and at least one further monomer chosen from the group consisting of n-butyl acrylate and ureidomethacrylate is used as copolymer (B).

13. The composition according to any of the preceding claims, wherein the (molar) ratio of copolymer (A) to copolymer (B) is in the range from 1:10 to 10:1, in particular in the range from 1:5 to 5:1.

14. The use of a composition according to any of the preceding claims as thickener.

15. The use of a composition according to any of the preceding claims as conditioning agent.

16. The use of a composition according to any of the preceding claims in cosmetic preparations.

17. A method of thickening cosmetic preparations in which 1 to 30% by weight, in particular 5 to 25% by weight, preferably 8 to 20% by weight, of a composition according to claim 1 are added to the preparation to be thickened.

## Revendications

1. Composition contenant
(A) un copolymère
(A-1) d'au moins un monomère éthyléniquement insaturé, copolymérisable par voie radicalaire, de la formule générale (I) :
Y-NR¹-C(V)-NHR² (I)
dans laquelle les substituants ont les significations suivantes :
Y = un radical éthyléniquement insaturé susceptible d'une polymérisation radicalaire,
V = O, S ou NH,
R¹, R² = indépendamment l'un de l'autre, H ou un groupe alkyle en C₁-C₈, ou les deux ensemble un groupe alkylène en C₂-C₄ formant pont, qui peut être substitué jusqu'à deux fois par un groupe alcoxy en C₁-C₄ et/ou un groupe hydroxy,
(A-2) d'au moins un monomère insaturé de la formule générale (II) :
X-C (O) CR⁷=CHR⁶ (II)
dans laquelle les substituants ont les significations suivantes :
X est choisi parmi le groupe des radicaux -OH, -OR⁸, NH₂, -NHR⁸, -N(R⁸)₂,
les radicaux R⁸ pouvant être choisis identiques ou différents parmi le groupe constitué de -H, des radicaux alkyle linéaires ou ramifiés en C₁-C₄₀, des groupes N,N-diméthylaminoéthyle, 2-hydroxyéthyle, 2-méthoxyéthyle, 2-éthoxyéthyle, hydroxypropyle, méthoxypropyle ou éthoxypropyle,
R⁷ et R⁶ sont, indépendamment l'un de l'autre, choisis parmi le groupe constitué de -H, de chaînes alkyliques linéaires ou ramifiées en C₁-C₈, de groupes méthoxy, éthoxy, 2-hydroxy-éthoxy, 2-méthoxyéthoxy et 2-éthoxyéthyle,
(B) au moins un autre copolymère différent de (A) à base
(B-1) d'au moins un monomère de la formule générale (III) : 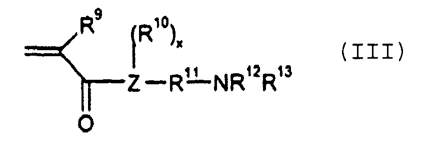 où
R⁹ = H, un groupe alkyle comportant 1 à 8 atomes de C,
R¹⁰ = H, méthyle,
R¹¹ = un groupe alkylène comportant 1 à 24 atomes de C, éventuellement substitué par un groupe alkyle en C₁-C₆,
R¹², R¹³ = radical alkyle en C₁-C₄₀,
Z = azote pour x = 1 ou oxygène pour x = 0,
et
(B-2) d'au moins un monomère éthyléniquement insaturé.

2. Composition suivant la revendication 1, **caractérisée en ce que**, comme copolymère (A), on met en oeuvre un copolymère d'au moins un monomère (A-1) et d'au moins deux monomères (A-2).

3. Composition suivant la revendication 1, **caractérisée en ce que**, comme monomère (A-1), on met en oeuvre un composé de la formule suivante (Ia) : 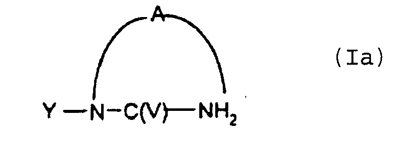 dans laquelle A = un groupe alkylène à 2 ou 3 termes, éventuellement à un groupe carbonyle.

4. Composition suivant la revendication 1, **caractérisée en ce que**, comme monomère (A-1), on met en oeuvre un composé de la formule (Ib) : 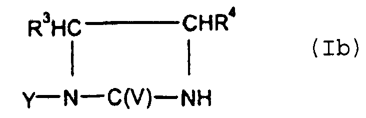 dans laquelle R³ et R⁴ représentent, indépendamment l'un de l'autre, H, -OH, -NH, un groupe alkyle en C₁-C₈.

5. Composition suivant la revendication 4, dans laquelle R³ et R⁴ = H.

6. Composition suivant l'une des revendications précédentes, **caractérisée en ce que**, comme monomère (A-1), on met en oeuvre un composé dans lequel V = O.

7. Composition suivant l'une des revendications précédentes, **caractérisée en ce que**, comme monomère (A-1), on met en oeuvre un composé de la formule (Ib) où R³ et R⁴ = H et V = O, et Y = CH₂=C(CH₃)-CO-O-(CH₂)₂-

8. Composition suivant l'une des revendications précédentes, **caractérisée en ce que** le radical Y éthyléniquement insaturé, susceptible d'une polymérisation radicalaire, représente un radical de la formule (IV) :
Y=CH₂=CR⁵-CO-W- (CH₂)ₙ- (IV),
dans laquelle
R⁵ = H, CH₃,
W = O, NH,
n = 2 à 8, en particulier 2 à 4.

9. Composition suivant l'une des revendications précédentes, **caractérisée en ce que**, comme monomère (B-1), on met en oeuvre au moins un monomère qui est choisi parmi le groupe constitué de (méth)acrylate de N,N-diméthylaminométhyle, de (méth)acrylate de N,N-diéthylaminométhyle, de (méth)acrylate de N,N-diméthylaminoéthyle, de (méth)acrylate de N,N-diéthylaminoéthyle, de (méth)acrylate de N,N-diméthylaminopropyle, de (méth)acrylate de N,N-diéthylaminopropyle.

10. Composition suivant l'une des revendications précédentes, **caractérisée en ce que**, comme monomère (B-1), on met en oeuvre du (méth)acrylate de N,N-diméthylaminopropyle.

11. Composition suivant l'une des revendications précédentes, **caractérisée en ce que**, comme copolymère (A), on met en oeuvre un copolymère de (A-1) uréidométhacrylate et de (A-2) au moins deux autres monomères choisis parmi le groupe constitué de l'acrylate de n-butyle, de l'acide acrylique et du méthacrylate de stéaryle.

12. Composition suivant l'une des revendications précédentes, **caractérisée en ce que**, comme copolymère (B), on met en oeuvre un copolymère de (B-1) (méth)acrylate de N,N-diméthylaminopropyle et d'au moins un autre monomère choisi parmi le groupe constitué de l'acrylate de n-butyle et de l'uréidométhacrylate.

13. Composition suivant l'une des revendications précédentes, **caractérisée en ce que** le rapport (molaire) entre le copolymère (A) et le copolymère (B) est de l'ordre de 1/10 à 10/1, en particulier de l'ordre de 1/5 à 5/1.

14. Utilisation d'une composition suivant l'une des revendications précédentes, comme agent épaississant.

15. Utilisation d'une composition suivant l'une des revendications précédentes, comme agent de conditionnement.

16. Utilisation d'une composition suivant l'une des revendications précédentes dans des préparations cosmétiques.

17. Procédé d'épaississement de préparations cosmétiques, dans lequel on ajoute à la préparation à épaissir 1 à 30 % en poids, en particulier 5 à 25 % en poids, de préférence 8 à 20 % en poids, d'une composition suivant la revendication 1.
